# FASCICULE DE BREVET EUROPEEN

(11) **EP 2 605 639 B1**
(45) Date de publication et mention de la délivrance du brevet: **02.04.2014**
(21) Numéro de dépôt: 11755411.3
(22) Date de dépôt: 11.08.2011
(51) Int. Cl.: A01G 11/00, F22B 1/18

(54) **MACHINE POUR LA STERILISATION DE LA COUCHE ARABLE DU SOL**
MASCHINE ZUR STERILISIERUNG DER MUTTERBODENSCHICHT
MACHINE FOR STERILIZING THE TOPSOIL OF THE GROUND

(30) Priorité: 17.08.2010 FR 1003386
(43) Date de publication de la demande: 26.06.2013
(73) Titulaire: Innovapeur, 66200 Montescot (FR)
(72) Inventeur: HERNANDEZ, Robert, 66000 Perpignan (FR)
(74) Mandataire: Cornuejols, Georges
(86) Numéro de dépôt international: PCT/FR2011/051904
(87) Numéro de publication internationale: WO 2012/022907

(56) Documents cités:
- EP-A1- 1 479 287
- WO-A1-02/07502
- WO-A1-2009/021877
- FR-A1- 2 902 285

## Description

### Domaine technique.

La présente invention est du domaine des matériels utilisés en agriculture en vue de stérilisation de la couche arable des sols à l'aide de vapeur surchauffée et se rapporte plus particulièrement à une machine de projection de jets de vapeur sur et dans la couche arable.

### État de la technique antérieure.

En vue de débarrasser la couche arable de la vermine qui peut l'infester, il est connu d'épandre des produits phytosanitaires sur et dans le sol et ce avant et après l'ensemencement. Si une telle technique présente l'avantage d'être d'une mise en oeuvre rapide, elle est cependant à l'origine de pollutions sévères et persistantes de l'environnement. De surcroît elles exigent des précautions et contraintes particulières, notamment le personnel en charge de l'épandage doit être correctement protégé par des combinaisons vestimentaires appropriées et après épandage, le matériel utilisé doit être soigneusement nettoyé. Par ailleurs, l'agent de nettoyage utilisé, typiquement de l'eau, doit être récupéré et stocké dans des réservoirs dédiés en vue d'un traitement de dépollution ultérieur, coûteux en soi.

On connaît également des traitements consistant non pas à épandre des produits phytosanitaires, mais à projeter de la vapeur d'eau surchauffée dans le sol afin d'utiliser le pouvoir stérilisant de la chaleur.

De telles techniques présentent l'avantage de n'engendrer aucune pollution, mais les machines utilisant ces techniques présentent l'inconvénient d'une mise en oeuvre et d'une exploitation relativement coûteuses. Ceci tient essentiellement au fait que les sources de production de vapeur dont sont équipées ces machines, sont d'un rendement relativement moyen et utilisent, pour assurer la production de vapeur surchauffée, un bouilleur utilisant la combustion d'énergie fossile, typiquement du gasoil, d'un coût de plus en plus important.

En outre, avec de telles machines, il a été observé une consommation importante d'eau qui implique la présence de réservoir de grande capacité induisant une augmentation notable de poids.

Enfin le rendement de ces machines, en termes de surface traitée par unité de temps, demeure trop en deçà de ce qui peut être obtenu par les traitements phytosanitaires de sorte que cette dernière méthode, malgré tous ses inconvénients, est encore à ce jour, largement utilisée.

On a cherché par le passé à résoudre les problèmes du rendement des machines de traitement par la vapeur. Ainsi on connaît une machine automobile dont la motorisation est constituée par un moteur thermique. Les gaz d'échappement sont récupérés et utilisés pour stériliser le sol. L'inconvénient de cette solution est l'introduction d'éléments polluants dans la couche arable.

On connaît du brevet FR 2 902 285 une machine utilisant en tant que source de production de vapeur un compresseur d'air étagé à taux de compression élevé propre à délivrer un flux d'air surchauffé apte d'une part, à assurer la vaporisation de l'eau et d'autre part, à transporter la vapeur d'eau formée, vers des buses de délivrance. Le principal intérêt de cette disposition est de s'affranchir de l'utilisation de bouilleurs et d'éviter l'introduction d'éléments polluants dans le sol, mais une telle machine transporte en plus du moteur, une masse supplémentaire constituée par le compresseur d'air.

On connaît également de la demande de brevet WO2009021877 un équipement à usage agricole pour l'injection dans le sol d'un fluide sous pression, et véhicule comportant un tel équipement.

Essentiellement, l'équipement installé à l'arrière du véhicule, est constitué par un tambour horizontal entraîné en rotation selon un axe horizontal transversal à la direction d'avancement du véhicule. Ce tambour creux est raccordé par un joint tournant à une unité de production de vapeur et comporte une série de bras radiaux creux portant des injecteurs de vapeur. Au cours de la rotation du tambour, les bras radiaux sont assujettis à pénétrer dans le sol pour y injecter la vapeur et pour participer à l'effort propulsif.

Un tel véhicule ne réalise pas une fonction d'ameublissement du sol facilitant la diffusion de la vapeur dans le sol. En outre dans la mesure où l'effort de propulsion du véhicule est produit en partie par les bras radiaux du tambour, ce dernier ne peut être entraîné que selon un sens dit en « avalant ».

Des machines de traitement des sols à l'aide de vapeur d'eau surchauffée sont également connues de la demande de brevet EP 1 479 287 et de la demande internationale WO0207502. Ces machines mettent en oeuvre des dents portant des buses d'injection de vapeur d'eau chaude connectées à une centrale de production de vapeur. Ces dents sont prévues pour pénétrer dans le sol afin d'y injecter la vapeur.

### Exposé de l'invention.

La présente invention a pour but de résoudre les inconvénients précédemment cités en mettant en oeuvre une machine automobile de traitement des sols par la vapeur d'eau, d'un rendement amélioré, selon la revendication 1.

À cet effet la machine automobile de traitement des sols par la vapeur selon l'invention, comprenant un groupe de propulsion pourvu d'un moteur thermique, un réservoir d'eau, et des moyens de production d'au moins un flux de vapeur d'eau surchauffée amené à une ou plusieurs buses de délivrance se caractérise essentiellement en ce que les moyens de production de vapeur surchauffée comprennent :
- une unité de pulvérisation en relation de communication avec le réservoir d'eau, ladite unité comprenant une chambre de pulvérisation dans laquelle est disposé un dispositif de pulvérisation pour y former un aérosol composé de fines particules d'eau,
- une unité apte à générer un flux d'air de transport amené à traverser ladite chambre de pulvérisation pour entraîner hors de cette chambre l'aérosol formé,
- une unité de chauffage pour élever la température de l'aérosol de façon que ce dernier soit transformé en vapeur.

La division en fines gouttelettes de l'eau permet d'en faciliter la vaporisation ultérieure et améliore considérablement le rendement de la machine en termes de valeur de surface traitée par unité de temps. En outre, la division en fines gouttelettes de l'eau permet une vaporisation optimale ce qui est un facteur de réduction de consommation d'eau.

Selon une autre caractéristique de l'invention, la chambre de pulvérisation est formée dans le réservoir, au-dessus du niveau maximal d'eau et la partie supérieure du réservoir est traversée par le flux d'air porteur, cette partie supérieure formant la chambre de mélange.

De préférence, selon une autre caractéristique de l'invention, le dispositif de pulvérisation comprend au moins une tête à ultrasons montée dans la partie supérieure du réservoir d'eau au-dessus du niveau maximal de liquide.

Avantageusement, selon une autre caractéristique de l'invention, le dispositif de chauffage du flux d'air comprend un échangeur de chaleur air/air doté d'une première voie traversée par les gaz d'échappement du moteur thermique et d'une seconde voie traversée par le mélange du flux d'air porteur et de l'aérosol, cette seconde voie étant en relation d'échange thermique avec la première voie au travers d'au moins une paroi d'échange thermique et en relation de communication étanche avec la chambre de pulvérisation.

L'intérêt d'une telle disposition est de récupérer l'énergie calorifique des gaz d'échappement pour chauffer le mélange flux d'air et aérosol afin de vaporiser l'eau tout en évitant la pollution de la couche arable par ces gaz d'échappement.

Selon une autre caractéristique de l'invention, l'échangeur de chaleur est disposé en aval de la chambre de pulvérisation, et auquel cas la seconde voie est également en relation de communication étanche avec la ou les buses de pulvérisation.

En variante, selon encore une autre caractéristique de l'invention, l'échangeur de chaleur est disposé en amont. Dans cette configuration, l'air porté est amené à haute température avant de pénétrer dans la chambre pulvérisation.

Selon une autre caractéristique de l'invention, la machine est dotée d'un dispositif d'ameublissement de la couche arable, ce dispositif étant équipé d'une structure support portant au moins une dent de travail de la terre pourvue dans sa partie active (partie prévue pour venir dans le sol et l'ameublir) d'au moins une buse de délivrance du flux d'air chaud chargé en vapeur d'eau surchauffée.

Une telle disposition permet, tout en ameublissant la couche arable, d'introduire la vapeur d'eau surchauffée dans l'épaisseur de cette dernière ce qui améliore grandement l'efficacité du traitement.

Selon une autre caractéristique de l'invention, la ou chaque buse de délivrance est en relation de communication avec un canal interne pratiqué dans la dent de travail de la terre, ce canal interne étant en relation de communication avec un second canal pratiqué dans la structure support de la dent, ce second canal étant en relation de communication par une conduite d'alimentation avec la sortie de la seconde voie du dernier échangeur de chaleur.

Selon une autre caractéristique de l'invention, la structure support de dent est un rotor entraîné par une transmission de mouvement accouplée à l'arbre de sortie du moteur thermique, un joint tournant étant disposé entre le second canal et la conduite d'alimentation, ledit rotor portant au moins une dent dont le canal interne est en relation de communication avec le second canal pratiqué dans la structure support, ce second canal étant axialement formé dans le rotor constituant cette structure.

Selon une autre caractéristique de l'invention, la machine comporte un second dispositif d'ameublissement de la terre ce dernier occupant une position amont par rapport au premier.

### Description sommaire des figures des dessins.

D'autres avantages buts et caractéristiques de l'invention apparaîtront à la lecture de la description d'une forme préférée de réalisation donnée à titre d'exemple non limitatif en se référant aux dessins annexés en lesquels :

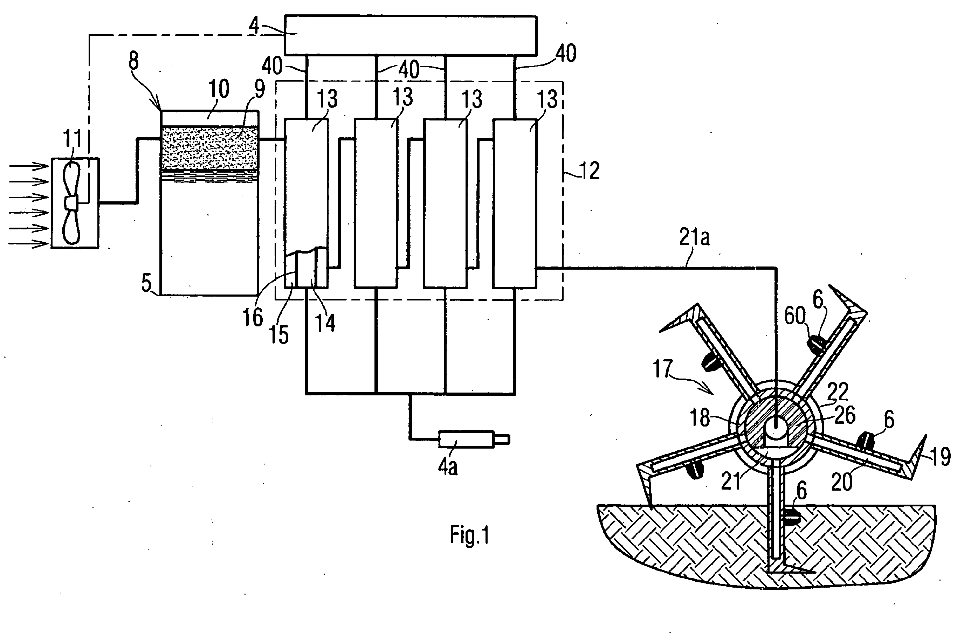

- la figure 1 est une vue schématique d'une machine selon l'invention,

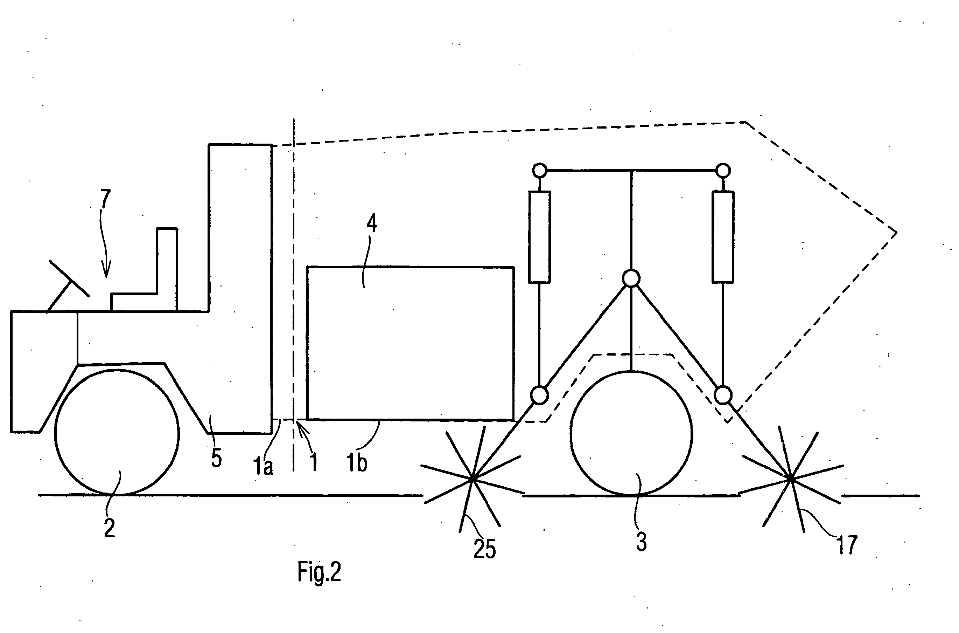

- la figure 2 est une vue de profil sous forme schématique d'une machine selon l'invention,

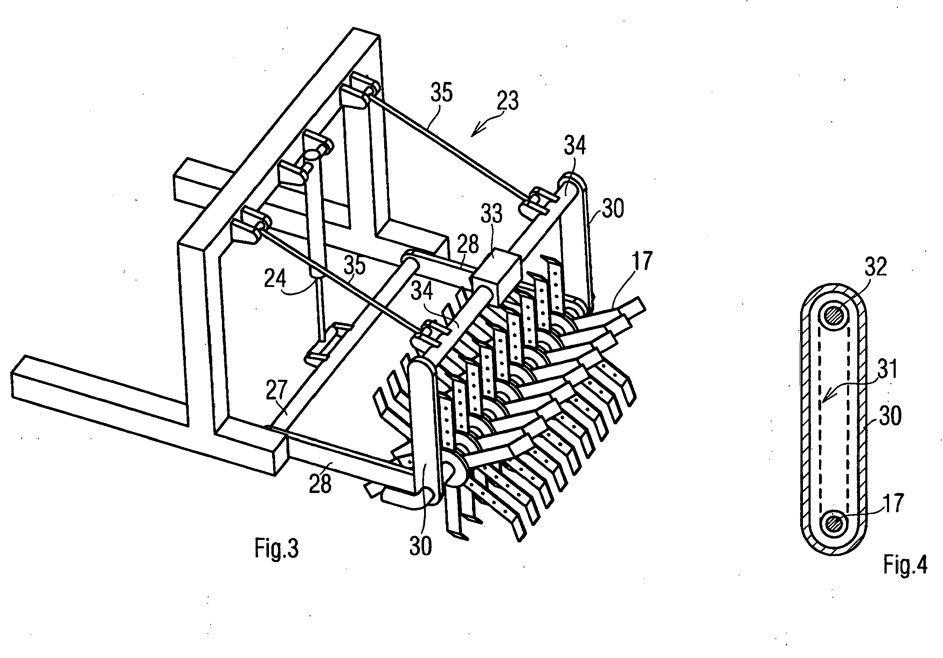

- la figure 3 est une vue en perspective montrant un système de relevage du rotor de travail de la terre,
- la figure 4 est une vue en coupe montrant un détail du mécanisme de relevage.

### Meilleure manière de réaliser l'invention.

Telle que représentée, la machine automobile selon l'invention comprend un châssis 1 monté sur des organes de roulement agencés en un train directeur 2 et un train moteur 3, ledit châssis 1 portant notamment un groupe de propulsion 4 formé préférentiellement d'un moteur thermique doté d'un arbre de sortie rotatif en prise avec une transmission de mouvement en relation d'accouplement avec le train moteur 3 de la machine.

Le châssis 1 est par ailleurs équipé d'un réservoir d'eau 5, de moyens de production de vapeur d'eau surchauffée en relation de communication d'une part avec le réservoir 5 et d'autre part avec une buse 6 de délivrance de vapeur d'eau dans la couche arable du sol à stériliser, cette buse 6 possédant un canal interne traversant 60 prévu pour être traversé par le flux de vapeur d'eau à injecteur dans le sol.

Préférentiellement, le châssis 1 comprend d'une part, une partie avant 1 a équipée du train directeur 2, du réservoir d'eau 5 et d'un poste de conduite 7 et d'autre part, une partie arrière 1 b articulée à la précédente selon un axe vertical, cette partie arrière 1 b étant équipée notamment du train moteur 3 et du groupe de propulsion 4.

Selon la forme préférée de réalisation, les moyens de production de vapeur d'eau comprennent :
- une unité de pulvérisation 8 en relation de communication avec le réservoir d'eau 5, ladite unité comprenant une chambre de pulvérisation 9 dans laquelle est disposé un dispositif de pulvérisation 10 pour y former un aérosol composé de fines particules d'eau,
- une unité 11 apte à générer un flux d'air de transport amené à traverser la chambre de pulvérisation 9 pour entraîner, hors de cette chambre, l'aérosol formé,
- une unité de chauffage 12 apte à élever la température de l'aérosol de façon que ce dernier soit transformé en vapeur.

L'unité de pulvérisation 8 est disposée de préférence dans le réservoir 5 au-dessus du niveau maximal d'eau et la chambre de pulvérisation 9 est formée dans le réservoir au-dessus du niveau maximal d'eau.

Le dispositif de pulvérisation 10 est fixé aux parois du réservoir 5 et est constitué de préférence par une ou plusieurs têtes aptes à délivrer des ultrasons sous l'effet desquels la couche superficielle d'eau dans le réservoir est pulvérisée en fines gouttelettes afin de former un aérosol.

L'unité 11 apte à générer le flux d'air de transport est constituée essentiellement d'une soufflante intégrant, dans une coque appropriée, un ventilateur actionné par le moteur thermique du groupe de propulsion 4 par l'intermédiaire d'une transmission de mouvement. Cette soufflante est en relation de communication par une conduite appropriée avec le volume de la chambre de pulvérisation 9. Plus particulièrement, cette conduite est connectée à un orifice d'entrée d'air formé dans la paroi de ladite chambre. À l'opposé de cet orifice d'entrée d'air, la chambre de pulvérisation 9 comporte un orifice de sortie par l'intermédiaire duquel elle est en relation de communication avec les buses de délivrance 6. On comprend donc que le flux d'air est amené à traverser la chambre de pulvérisation 9 et entraîner hors de cette chambre lors de son passage l'aérosol formé par le moyen de pulvérisation.

Dans la forme préférée de réalisation, l'unité de chauffage 12 est disposée en aval de la chambre de pulvérisation 9 et est en relation de communication d'une part avec ladite chambre 9 et d'autre part avec les buses de délivrance 6 et plus particulièrement avec le canal interne 60 de chacune de ces dernières. Cette unité de chauffage 12 est traversée par le flux d'air chargé en aérosol et a pour but de céder au mélange formé, les calories nécessaires à la vaporisation des gouttelettes d'eau composant l'aérosol.

Dans la forme préférée de réalisation, l'unité de chauffage 12 comprend au moins un échangeur de chaleur 13 du type air /air doté d'une première voie 14 traversée par les gaz d'échappement de l'une au moins chambre de travail du moteur thermique et d'une seconde voie 15 traversée par le flux d'air porteur et l'aérosol. Cette seconde voie 15 est en relation d'échange thermique avec la première voie 14 au travers d'au moins une paroi d'échange thermique 16 et est en relation de communication étanche tant avec la chambre de pulvérisation 9 qu'avec les buses de délivrance 6.

Préférentiellement, la machine comporte autant d'échangeurs de chaleur 13 que le moteur thermique présente de chambres de travail, la pipe d'échappement de chaque chambre de travail étant connectée par une conduite 40 appropriée à la première voie 14 de l'échangeur thermique associé. Ainsi chaque chambre de travail alimente un échangeur de chaleur 13 et un seul, et la sortie de la première voie 14 de chaque échangeur de chaleur 13 est connectée à un pot d'échappement 4a commun. Les échangeurs de chaleur par leurs secondes voies 15 sont connectés en série. De cette manière, le flux d'air porteur chargé en eau pulvérisée et en vapeur d'eau passe successivement aux travers des différents échangeurs de chaleur 13 ce qui augmente graduellement la température de ce mélange et l'amène au-dessus de la température de vaporisation de l'eau.

Ainsi, en sortie des échangeurs de chaleur, 13 sera obtenu un flux d'air surchauffé chargé en vapeur d'eau surchauffée. À titre d'exemple purement indicatif, la température obtenue en sortie des échangeurs de chaleur est de l'ordre de 280 degrés Celsius.

La sortie de la seconde voie 15 du dernier échangeur de chaleur est en relation de communication au travers d'une conduite 21 a avec les buses de délivrance 6.

Dans la forme préférée de réalisation, les buses de délivrance 6 sont portées par un dispositif 17 d'ameublissement de la couche arable du sol. Ce dispositif 17 est constitué par une structure support 18 portant plusieurs dents 19 de travail de la terre pourvues chacune, dans leurs parties actives, d'au moins une buse de délivrance 6.

Dans la forme préférée de réalisation, chaque dent 19 comporte un canal interne 20 et la ou chaque buse de délivrance 6, portée par cette dent est par son canal interne traversant 61, en relation de communication avec le canal 20. Ce canal interne 20 est en relation de communication avec un second canal 21 pratiqué dans la structure support 18 de la dent 19, ce second canal 21 étant en relation de communication avec la sortie de la seconde voie du dernier échangeur de chaleur par l'intermédiaire d'une conduite d'alimentation 21 a.

De préférence, le support 18 de dent est un rotor entraîné par une transmission de mouvement accouplée à l'arbre de sortie du moteur thermique. Le canal 21 est formé dans le rotor selon l'axe longitudinal de ce dernier et se présente sous la forme d'un alésage. Un joint tournant 22 est disposé entre le second canal 21 et la conduite d'alimentation.

Selon une forme préférée de réalisation, le dispositif d'ameublissement 17 est monté sur un équipage 23 mobile entre une position haute ou position de repos selon laquelle le dispositif d'ameublissement est disposé au-dessus et à écartement du sol et une position basse ou position active selon laquelle ce dispositif d'ameublissement pénètre dans le sol pour l'ameublir. L'équipage mobile 23 sera associé à un organe moteur 24 de relevage, du genre vérin pneumatique ou hydraulique par exemple. Par la commande de cet organe moteur, l'équipage mobile 23 sera soit abaissé soit relevé.

Le rotor de travail de la terre 17 pourra être à axe de rotation horizontal et dans ce cas le joint tournant 22 sera associé à un distributeur 26 connu en soi, apte à alimenter chaque dent en air chaud et vapeur lors de son trajet dans la terre et à interrompre cette alimentation lorsque la dent est au-dessus du sol. En raison de cette disposition seront évitées toutes pertes inutiles de vapeur.

L'équipage mobile 23 est constitué par une structure pivotante formée d'un arbre horizontal 27 engagé en rotation dans deux paliers d'extrémité solidaires du châssis, ledit arbre 27 portant deux bras radiaux 28, latéraux, parallèles, dotés chacun à distance de l'arbre, d'un palier support dans lequel est engagé le rotor 17. L'arbre horizontal 27 est doté d'un bras de levier 29, radial, auquel est articulée l'extrémité de la tige du vérin de relevage 24 articulé par son corps au châssis de la machine. Aux bras radiaux 28, coaxialement au rotor 17, sont articulés deux flasques rigides creux 30, aptes chacun à recevoir une transmission de mouvement 31 en prise avec un demi-arbre de transmission 32 actionné par un organe moteur 33 porté par une barre entretoise 34 réunissant rigidement l'un à l'autre les deux flasques creux, cette barre entretoise 34 étant creuse pour recevoir les demi-arbre de transmission 32. Cette barre 34 ou les flasques 30 reçoit ou reçoivent en articulation deux bielles 35 articulées par ailleurs au châssis de la machine. Les bielles 35, les flasques 30, les bras radiaux 28 et le châssis forment une structure à parallélogramme déformable.

Chaque transmission 31 de mouvement entre le demi-arbre correspondant 32 et le rotor 17 sera constituée par deux pignons dentés respectivement calés sur le demi-arbre et sur le rotor et par une chaîne à maillons articulés engrenée avec les deux pignons.

En variante le dispositif d'ameublissement de la terre est constitué par au moins une herse rotative à axe de rotation vertical. Cette herse rotative comprend un rotor à axe de rotation vertical auquel sont fixées les dents 19 de travail de la terre. Selon cette disposition, les dents 19 du rotor travaillent simultanément le sol et la vapeur est distribuée simultanément aux diverses buses des différentes dents. Ainsi le rotor est exempt de distributeur.

Avantageusement, en avant du dispositif d'ameublissement 17, la machine selon l'invention comporte un second dispositif d'ameublissement 25 sous forme de rotor avec dents. Ce dispositif a pour but d'effectuer un ameublissement préparatoire afin de favoriser ultérieurement la diffusion de la vapeur dans la couche arable du sol.

## Revendications

1. Machine pour la stérilisation d'une couche arable avant ensemencement, par injection de vapeur d'eau surchauffée dans cette dernière, comprenant un châssis (1) monté sur des organes de roulement agencés en trains et sur lequel châssis sont installés un groupe de propulsion (4) apte à délivrer un couple d'entraînement communiqué à l'un des trains de roues au moins, un réservoir d'eau (5), des moyens de production de vapeur d'eau surchauffée et de transport de cette dernière vers au moins une buse de délivrance (6) **caractérisée en ce que** les moyens de production de vapeur d'eau surchauffée, comprennent :
- une unité de pulvérisation (8) en relation de communication avec le réservoir d'eau, ladite unité comprenant une chambre de pulvérisation (9) dans laquelle est disposé un dispositif de pulvérisation (10) pour y former un aérosol composé de fines particules d'eau,
- une unité (11) apte à générer un flux d'air de transport amené à traverser ladite chambre de pulvérisation (9) pour entraîner hors de cette chambre l'aérosol formé,
- une unité de chauffage (12) pour élever la température de l'aérosol de façon que ce dernier soit transformé en vapeur.

2. Machine selon la revendication 1, **caractérisée en ce que** la chambre de pulvérisation (9) est formée dans le réservoir (5) au-dessus du niveau maximal d'eau.

3. Machine selon la revendication précédente, **caractérisée en ce que** le dispositif de pulvérisation (10) comprend au moins une tête à ultrasons.

4. Machine selon l'une quelconque des revendications précédentes **caractérisé en ce que** le groupe de propulsion comprend un moteur thermique et que l'unité de chauffage (12) comprend au moins un échangeur de chaleur air/air (13) doté d'une première voie (14) traversée par les gaz d'échappement de l'une au moins chambre de travail du moteur thermique et d'une seconde voie (15) traversée par le flux d'air de transport, cette seconde voie étant en relation d'échange thermique avec la première voie au travers d'au moins une paroi d'échange thermique (16) et étant en relation de communication étanche avec la chambre de pulvérisation (9).

5. Machine, selon la revendication précédente, **caractérisée en ce que** l'échangeur de chaleur (13) est installé en aval de la chambre de pulvérisation (9) et est traversé par le mélange flux d'air de transport et aérosol, la seconde voie (15) de l'échangeur de chaleur (13) étant en relation de communication avec les buses de délivrance (6).

6. Machine selon la revendication précédente, **caractérisée en ce qu'**elle comporte autant d'échangeurs de chaleur (13) que le moteur thermique présente de chambres de travail, que chaque chambre de travail alimente un échangeur de chaleur (13) et un seul qui lui est propre, que la pipe d'échappement de chaque chambre de travail est connectée par une conduite appropriée (40) à la première voie (14) de l'échangeur thermique associé, et que les échangeurs de chaleur (13), par leurs secondes voies (15), sont connectées en série.

7. Machine selon l'une quelconque des revendications précédentes, **caractérisée en ce qu'**elle est dotée d'un dispositif d'ameublissement (17) de la couche arable, ce dispositif (17) étant équipé d'une structure support (18) portant au moins une dent (19) de travail de la terre pourvue dans sa partie active d'au moins une buse de délivrance (6).

8. Machine selon les revendications 6 et 7 prises ensemble, **caractérisée en ce que** la ou chaque buse de délivrance (6) est en relation de communication avec un canal interne (20) pratiqué dans la dent (19) de travail de la terre, ce canal interne (20) étant en relation de communication avec un second canal (21) pratiqué dans la structure support de la dent, ce second canal (21) étant en relation de communication par une conduite d'alimentation (21a) avec la sortie de la seconde voie (15) du dernier échangeur de chaleur (13).

9. Machine selon la revendication précédente, **caractérisée en ce que** la structure support (18) de dent est un rotor entraîné par une transmission de mouvement accouplée à l'arbre de sortie du moteur thermique, un joint tournant (22) étant disposé entre le second canal et la conduite d'alimentation, ledit rotor portant au moins une dent (19) dont le canal interne (20) est en relation de communication avec le second canal (21) pratiqué dans la structure support, ce second canal (21) étant axialement formé dans le rotor constituant cette structure.

10. Machine selon la revendication précédente, **caractérisée en ce que** le rotor (18) est à axe de rotation horizontal.

11. Machine selon la revendication 8, **caractérisée en ce que** la structure support (18) de dent est une herse rotative à axe de rotation vertical.

12. Machine selon l'une quelconque des revendications 7 à 11, **caractérisée en ce qu'**elle comporte un second dispositif d'ameublissement (25) monté en avant du premier.

## Patentansprüche

1. Maschine zur Sterilisierung einer Ackerbodenschicht vor dem Aussäen, indem überhitzter Wasserdampf in dieselbe eingeleitet wird, umfassend ein Fahrgestell (1), das auf Rollelementen montiert ist, welche fahrwerkartig angeordnet sind, wobei auf dem Fahrgestell ein Antriebsstrang (4), der dazu geeignet ist, ein Antriebsdrehmoment bereitzustellen, welches auf mindestens eines der Räderfahrwerke übertragen wird, sowie ein Wasserbehälter (5) und Mittel zur Erzeugung von überhitztem Wasserdampf und zur Beförderung desselben in Richtung mindestens einer Abgabedüse (6) angeordnet sind, **dadurch gekennzeichnet, dass** die Mittel zur Erzeugung von überhitztem Wasserdampf Folgendes umfassen:
- eine Sprüheinheit (8), die mit dem Wasserbehälter in Verbindung steht, wobei die Einheit eine Sprühkammer (9) umfasst, in welcher eine Sprühvorrichtung (10) angeordnet ist, um dort ein Aerosol zu bilden, das aus feinen Wasserpartikeln besteht,
- eine Einheit (11), die dazu geeignet ist, einen Förderluftstrom zu erzeugen, der dazu bestimmt ist, die Sprühkammer (9) zu durchströmen, um das gebildete Aerosol aus diseer Kammer hinauszubefördern,
- eine Heizeinheit (12), um die Temperatur des Aerosols derart zu erhöhen, dass dieses in Dampf umgewandelt wird.

2. Maschine nach Anspruch 1, **dadurch gekennzeichnet, dass** die Sprühkammer (9) in dem Behälter (5) oberhalb des höchstmöglichen Wasserstandes ausgebildet ist.

3. Maschine nach dem vorhergehenden Anspruch, **dadurch gekennzeichnet, dass** die Sprühvorrichtung (10) mindestens einen Ultraschallkopf umfasst.

4. Maschine nach ein beliebigen der vorhergehenden Ansprüche, **dadurch gekennzeichnet, dass** der Antriebsstrang einen Verbrennungsmotor umfasst und dass die Heizeinheit (12) mindestens einen Luft/Luft-Wärmetauscher (13) umfasst, der mit einer ersten Leitung (14) versehen ist, durch welche die Abgase aus dem mindestens einem Arbeitszylinder des Verbrennungsmotors strömen, und mit einer zweiten Leitung (15), durch welche der Förderluftstrom strömt, wobei diese zweite Leitung über mindestens eine Wärmeaustauschwand (16) in Wärmeaustauschverbindung mit der ersten Leitung steht und wobei sie unter Abdichtung nach außen mit der Sprühkammer (9) verbunden ist.

5. Maschine nach dem vorhergehenden Anspruch, **dadurch gekennzeichnet, dass** der Wärmetauscher (13) stromabwärts der Sprühkammer (9) angeordnet ist und von der Mischung aus dem Förderluftstrom und dem Aerosol durchströmt wird, wobei die zweite Leitung (15) des Wärmetauschers (13) mit den Abgabedüsen (6) in Verbindung steht.

6. Maschine nach dem vorhergehenden Anspruch, **dadurch gekennzeichnet, dass** sie ebenso viele Wärmetauscher (13) aufweist wie der Verbrennungsmotor Arbeitszylinder hat, dass jeder Arbeitszylinder ein Wärmetauscher (13) speist, und zwar einen einzigen, der ihm zugeordnet ist, dass das Auspuffrohr jedes Arbeitszylinders mittels einer geeigneten Rohrleitung (40) an die erste Leitung (14) des zugeordneten Wärmetauschers angeschlossen ist, und dass die Wärmetauscher (13) über ihre zweiten Leitungen (15) in Reihe geschaltet sind.

7. Maschine nach einem beliebigen der vorhergehenden Ansprüche, **dadurch gekennzeichnet, dass** sie mit einer Vorrichtung (17) zur Auflockerung der Ackerbodenschicht versehen ist, wobei diese Vorrichtung (17) mit einer Trägerstruktur (18) ausgestattet ist, die mindestens einen Zahn (19) trägt, der zur Bodenbearbeitung dient und in seinem Wirkbereich mit mindestens einer Abgabedüse (6) versehen ist.

8. Maschine nach beiden der Ansprüchen 6 und 7, **dadurch gekennzeichnet, dass** die oder jede der Abgabedüse(n) (6) mit einem inneren Kanal (20) in Verbindung steht, der durch den Bodenbearbeitungszahn (19) führt, wobei der innere Kanal (20) mit einem zweiten Kanal (21) in Verbindung steht, der durch die Trägerstruktur des Zahnes führt, wobei der zweite Kanal (21) über eine Speiserohrleitung (21a) mit dem Ausgang der zweiten Leitung (15) des letzten Wärmetauschers (13) in Verbindung steht.

9. Maschine nach dem vorhergehenden Anspruch, **dadurch gekennzeichnet, dass** es sich bei der Zahnträgerstruktur (18) um einen Rotor handelt, der derart an die Abtriebswelle des Verbrennungsmotors gekoppelt ist, dass er von deren Bewegung angetrieben wird, wobei eine Drehverbindung (22) zwischen dem zweiten Kanal und der Speiserohrleitung angeordnet ist, wobei der Rotor mindestens einen Zahn (19) trägt, dessen innerer Kanal (20) mit dem zweiten Kanal (21) in Verbindung steht, welcher durch die Trägerstruktur führt, wobei dieser zweite Kanal (21) axial in dem Rotor ausgebildet ist, welcher diese Struktur bildet.

10. Maschine nach dem vorhergehenden Anspruch, **dadurch gekennzeichnet, dass** der Rotor (18) eine horizontale Drehachse hat.

11. Maschine nach Anspruch 8, **dadurch gekennzeichnet, dass** es sich bei der Zahnträgerstruktur (18) um eine Kreiselegge mit vertikaler Drehachse handelt.

12. Maschine nach einem beliebigen der Ansprüche 7 bis 11, **dadurch gekennzeichnet, dass** sie eine zweite Auflockemngsvorrichtung (25) aufweist, die vor der ersten angebracht ist.

## Claims

1. Machine for sterilizing topsoil before sowing, by injection of superheated water vapor in the topsoil, comprising a frame (1) that is mounted on rolling elements arranged in trains and on which frame are installed a propulsion set (4) that is capable of delivering a drive torque communicated to at least one of the wheel trains, a water tank (5), und means for producing superheated water vapor und for conveying the latter to at least one supply nozzle (6), **characterized in that** the means for producing superheated water vapor comprise:
- A spraying unit (8) in communication with the water tank, whereby said unit comprises a spraying chamber (9) in which a spraying device (10) is placed to form an aerosol that consists of fine water particles,
- A unit (11) that is capable of generating a stream of conveying air that is run through said spraying chamber (9) to entrain the formed aerosol outside of this chamber,
- A heating unit (12) for raising the temperature of the aerosol in such a way that the latter is transformed into steam.

2. Machine according to Claim 1, wherein the spraying chamber (9) is formed in the tank (5) above the maximum water level.

3. Machine according to the preceding claim, wherein the spraying device (10) comprises at least one ultrasonic head.

4. Machine according to any of the preceding claims, wherein the propulsion set comprises an internal combustion engine and the heating unit (12) comprises at least one air/air heat exchanger (13) that is equipped with a first path (14) through which the exhaust gases from at least one working chamber of the internal combustion engine pass and a second path (15) through which the conveyor air stream passes, with this second path being in heat exchange with the first path through at least one heat exchange wall (16) and in airtight communication with the spraying chamber (9).

5. Machine, according to the preceding claim, wherein the heat exchanger (13) is installed downstream from the spraying chamber (9) and through which the mixture of conveyor air stream and aerosol passes, with the second path (15) of the heat exchanger (13) being in communication with the supply nozzles (6).

6. Machine according to the preceding claim, wherein it comprises as many heat exchangers (13) as the internal combustion engine has working chambers, wherein each working chamber supplies one heat exchanger (13) and one only that is specific thereto, wherein the exhaust pipe of each working chamber is connected by a suitable pipe (40) to the first path (14) of the associated heat exchanger, and wherein the heat exchangers (13) are connected in series by their second paths (15).

7. Machine according to any of the preceding claims, wherein it is equipped with a device (17) for loosening topsoil, with this device (17) being equipped with a support structure (18) that carries at least one earth-working tooth (19) equipped in its active part with at least one supply nozzle (6).

8. Machine according to the preceding claim, wherein the supply nozzle (6) or each supply nozzle (6) is in communication with an inner channel (20) made in the earth-working tooth (19), with this inner channel (20) being in communication with a second channel (21) made in the support structure of the tooth, this second channel (21) being in communication via a feed pipe (21 a) with the outlet of the second path (15) of the last heat exchanger (13).

9. Machine according to the preceding claim, wherein the tooth support structure (18) is a rotor driven by a movement transmission coupled to the output shaft of the internal combustion engine, a rotary joint (22) being placed between the second channel and the feed pipe, with said rotor carrying at least one tooth (19) whose inner channel (20) is in communication with the second channel (21) made in the support structure, with this second channel (21) being axially formed in the rotor that constitutes this structure.

10. Machine according to the preceding claim, wherein the rotor (18) has a horizontal axis of rotation.

11. Machine according to Claim 8, wherein the tooth support structure (18) is a rotary harrow with a vertical axis of rotation.

12. Machine according to any of Claims 7 to 11, wherein it comprises a second loosening device (25), mounted in front of the first device.
